# EUROPÄISCHE PATENTSCHRIFT

(11) **EP 2 531 449 B1**
(45) Veröffentlichungstag und Bekanntmachung des Hinweises auf die Patenterteilung: **28.10.2015**
(21) Anmeldenummer: 11700448.1
(22) Anmeldetag: 20.01.2011
(51) Int. Cl.: C01G 99/00, A61K 51/02, G21G 1/04, G21G 1/10, G21G 1/00

(54) **VERFAHREN UND VORRICHTUNG ZUR PRODUKTION VON 99mTc**
METHOD AND DEVICE FOR PRODUCING 99mTc
PROCÉDÉ ET DISPOSITIF DE PRODUCTION DE 99mTc

(30) Priorität: 01.02.2010 DE 102010006435
(43) Veröffentlichungstag der Anmeldung: 12.12.2012
(73) Patentinhaber: Siemens Aktiengesellschaft, 80333 München (DE)
(72) Erfinder: BAURICHTER, Arnd, DK-5260 Odense (DK); HEID, Oliver, 91052 Erlangen (DE); HUGHES, Timothy, 91056 Erlangen (DE)
(86) Internationale Anmeldenummer: PCT/EP2011/050728
(87) Internationale Veröffentlichungsnummer: WO 2011/092102

(56) Entgegenhaltungen:
- EP-A2- 2 242 062
- US-A1- 2007 160 176
- B. GUÉRIN ET AL: "Comparing cyclotron-produced Tc-99m with generator produced Tc-99m", NUCLEAR MEDICINE AND BIOLOGY, ELSEVIER, NY, US, Bd. 37, Nr. 6, 1. August 2010 (2010-08-01) , Seiten 716-717, XP027209959, ISSN: 0969-8051 [gefunden am 2010-08-13]
- TAKÁCS S ET AL: "Evaluation of proton induced reactions on 100Mo: New cross sections for production of 99mTc and 99Mo", JOURNAL OF RADIOANALYTICAL AND NUCLEAR CHEMISTRY, SPRINGER, Bd. 257, Nr. 1, 1. Juli 2003 (2003-07-01), Seiten 195-201, XP002599949, ISSN: 0236-5731

## Beschreibung

Die Erfindung betrifft ein Verfahren und eine Vorrichtung zur Produktion von ^{99m}Tc. ^{99m}Tc wird unter anderem bei der medizinischen Bildgebung eingesetzt, zum Beispiel bei der SPECT-Bildgebung.

Ein handelsüblicher ^{99m}Tc-Generator ist ein Gerät zur Extraktion des metastabilen Isotops ^{99m}Tc aus einer Quelle, die zerfallendes ⁹⁹Mo enthält.

⁹⁹Mo seinerseits wird meistens in einem Verfahren gewonnen, das hochangereichertes Uran ²³⁵U als Target verwendet. Durch Bestrahlung des Targets mit Neutronen entsteht ⁹⁹Mo als Spaltprodukt. Aufgrund internationaler Abkommen wird es jedoch in Zukunft zunehmend schwieriger werden, Reaktoren mit hochangereichertem Uran zu betreiben, was zu einem Engpass bei der Lieferung von Radionuklide für die SPECT-Bildgebung führen kann.

Es ist daher die Aufgabe der Erfindung, ein Verfahren und eine Vorrichtung zur alternativen Produktion von ^{99m}Tc anzugeben.

Die Aufgabe der Erfindung wird gelöst durch die Merkmale der unabhängigen Ansprüche. Vorteilhafte Weiterbildungen der Erfindung finden sich in den Merkmalen der abhängigen Ansprüche.

Das erfindungsgemäße Verfahren zur Produktion von ^{99m}Tc umfasst folgende Schritte:
- Bereitstellen einer Lösung mit ¹⁰⁰Mo-Molybdat-Ionen,
- Bereitstellen eines Protonenstrahls mit einer Energie, die geeignet ist, bei Bestrahlung von ¹⁰⁰Mo-Molybdat-Ionen eine ¹⁰⁰Mo (p, 2n) ^{99m}Tc-Kernreaktion zu induzieren,
- Bestrahlen der Lösung mit dem Protonenstrahl und Induktion einer ¹⁰⁰Mo (p, 2n) ^{99m}Tc-Kernreaktion,
- Anwenden eines Extraktionsverfahrens zur Extraktion des ^{99m}Tc aus der Lösung.

Das ^{99m}Tc wird also direkt anhand einer Kernreaktion gewonnen, die durch Wechselwirkung des Protonenstrahls mit den Molybdän-Atomen stattfindet, nach der Gleichung ¹⁰⁰Mo(p,2n) ^{99m}Tc. Die Energie des Protonenstrahls ist größer als 20 MeV und befindet sich damit in einem Bereich, in dem der Wirkungsquerschnitt für die genannte Kernreaktion liegt. Damit können ^{99m}Tc Atome in einer für die Produktion von ^{99m}Tc ausreichenden Anzahl gewonnen werden. Dadurch, dass die Molybdän-Atome als Molybdat-Ionen in einer Lösung vorliegen, kann anschließend das entstandene ^{99m}Tc auf einfache Weise mithilfe eines Extraktionsverfahrens aus der Lösung extrahiert werden. Das extrahierte ^{99m}Tc kann dann für verschiedene Zwecke verwendet werden, insbesondere zur Herstellung eines Radionuklids für die SPECT-Bildgebung.

Der Protonenstrahl wird auf eine Energie von mindestens 20 MeV beschleunigt. Vorzugsweise wird der Partikelstrahl auf eine Energie von 20 MeV bis 25 MeV beschleunigt. Durch Begrenzung der maximalen Energie auf maximal 35 MeV, insbesondere auf 30 MeV und höchst insbesondere auf 25 MeV wird vermieden, dass durch eine zu hohe Energie des Partikelstrahls Kernreaktionen ausgelöst werden, die zu unerwünschten Reaktionsprodukten führen, z.B. zu anderen Tc-Isotopen als ^{99m}Tc, die dann wieder einen zusätzlichen Schritt erfordern, durch dem die unerwünschten Reaktionsprodukte wieder entfernt werden. Die Kammer, in der die Lösung mit Molybdat-Ionen gehalten wird, kann derart beschaffen bzw. dimensioniert sein, dass der austretende Partikelstrahl eine Energie von mindestens 10 MeV aufweist. Auf diese Weise kann der Energiebereich des Protonenstrahls in einem Bereich gehalten werden, in dem die auftretenden Kernreaktionen kontrollierbar bleiben und in dem unerwünschte Reaktionsprodukte lediglich in einem akzeptablen Ausmaß auftreten.

Die Beschleunigung von Protonen auf die genannte Energie benötigt üblicherweise lediglich eine einzige Beschleunigereinheit mittlerer Größe, die auch lokal eingesetzt und installiert werden kann. ^{99m}Tc lässt sich mit dem beschriebenen Verfahren lokal in der Nähe bzw. im Umfeld des gewünschten Einsatzortes, beispielsweise im Umfeld eines Krankenhauses, erzeugen. Im Gegensatz zu herkömmlichen, nicht lokalen Produktionsmethoden, die mit dem Einsatz von großen Anlagen wie in Kernreaktoren und dem damit verbundenen Verteilungsproblem einhergehen, löst eine lokale Herstellung viele Probleme. Nuklearmedizinische Abteilungen können ihren Workflow unabhängig voneinander planen und sind nicht auf eine aufwändige Logistik und Infrastruktur angewiesen.

In einer Ausführungsform kann das Extraktionsverfahren ein Flüssig-Flüssigextraktionsverfahren sein, insbesondere unter Verwendung von Methylethylketon.

Dieses Extraktionsverfahren bietet sich an, da ^{99m}Tc in einer Lösung vorliegt. Das ^{99m}Tc löst sich in Methylethylketon, wobei die Molybdat-Ionen weiterhin in der wässrigen Lösung verbleiben. Auf diese Weise kann das ^{99m}Tc von dem ¹⁰⁰Mo getrennt werden. Das ^{99m}Tc geladene Methylethylketon kann z.B. getrocknet werden, sodass anschließend das ^{99m}Tc z.B. für die Herstellung eines Radiopharmakons verwendet werden kann.

In einer Ausführungsform können die nach Extraktion des ^{99m}Tc verbleibenden gelösten ¹⁰⁰Mo-Molybdat-Ionen der zu bestrahlenden Lösung wieder zugeführt werden, beispielsweise in einem geschlossenen Kreislauf. So wird sichergestellt, dass das Ausgangsmaterial, nämlich die ¹⁰⁰Mo-Molybdat-Ionen besonders effizient verwendet werden.

In einer Ausführungsform ist die Lösung mit ¹⁰⁰Mo-Molybdat-Ionen eine Lösung eines ¹⁰⁰Mo-Molybdat-Salzes, wobei in der Lösung durch Bestrahlen mit dem Protonenstrahl bei den Kationen des ¹⁰⁰Mo-Molybdat-Salzes eine Kernreaktion induziert wird, welche in mindestens ein Kationen-Endprodukt mündet, insbesondere in ein Kationen-Endprodukt, das in der ursprünglich zu bestrahlenden Lösung nicht vorhanden war, das ein Ion ist, das instabil ist und/oder das potentiell schädlich für einen menschlichen Körper ist. Der Terminus "Kationen-Endprodukt" bedeutet nicht zwangsläufig, dass das Endprodukt ein Kation sein muss, es kennzeichnet lediglich, dass das Endprodukt aus den Kationen des Salzes herrührt.

In diesem Fall können die verbleibenden, gelösten ¹⁰⁰Mo-Molybdat-Ionen nach Extraktion des ^{99m}Tc der bestrahlenden Lösung wieder zugeführt werden, wobei das mindestens eine Kationen-Endprodukt vor Zuführung entfernt wird, insbesondere unter Verwendung eines Ionentauschers.

Diese Ausführungsform kann den Vorteil aufweisen, dass die der zu bestrahlenden Lösung wieder zugeführte Lösung keine Bestandteile enthält, die bei erneuter Bestrahlung mit dem Protonenstrahl zu weiteren Bestrahlungsprodukten führen würden, die sich von den Kationen-Endprodukten unterscheiden. Es kann dann z.B. vermieden werden, dass der Lösung Kationen-Endprodukte zugeführt würden, die bei Bestrahlung zu weiteren, neuen Kernreaktionen führen würden. Hierdurch lässt sich erreichen, dass trotz Rückführung der Molybdat-Ionen unkontrollierte oder unüberschaubare Kernreaktionen vermieden werden.

In einer Ausführungsform kann das extrahierte ^{99m}Tc von Verunreinigungen durch das Kationen-Endprodukt gereinigt werden, insbesondere unter Verwendung eines Ionenaustauschers.

Auf diese Weise können z.B. potentiell unerwünschte Bestandteile der extrahierten ^{99m}Tc-Lösung vor einer weiteren Verarbeitung entfernt werden. So können beispielsweise potentielle, für einen menschlichen Körper toxische Stoffe vor Herstellung des Radionuklids entfernt werden oder andere Radionuklide mit einer anderen Halbwertszeit.

In einer Ausführungsvariante umfasst das ¹⁰⁰Mo-Molybdat-Salz ⁶Li₂¹⁰⁰MoO₄. Das ⁶Li zerfällt durch die Kernreaktion ⁶Li(p, 3He)⁴H zu ⁴H, das seinerseits sofort zu Tritium zerfällt.

Bei Verwendung von ⁷Li würde durch den Beschuss mit dem Protonstrahl die Reaktion ⁷Li(p,n) ⁷Be ausgelöst werden, wobei das ⁷Be wieder entfernt werden müsste. Die Verwendung von ⁶Li vermeidet das.

Auf diese Weise entsteht kein Kationen-Endprodukt, das bei erneuter Bestrahlung mit dem Protonenstrahl zu einer unkontrollierten Kette von Kernreaktionen führen würde. Auf die Reinigungsstufe, mit der das entstehende Kationen-Endprodukt entfernt wird, kann gegebenenfalls verzichtet werden.

In einer anderen Ausführungsvariante umfasst das ¹⁰⁰Mo-Molybdat-Salz Na₂¹⁰⁰MoO₄. Das mindestens eine Kationen-Endprodukt umfasst dabei ¹⁸F. Das natürlich vorkommende ²³Na wird durch den Beschuss mit dem Protonstrahl durch die Reaktion ²³Na(p,n) ²³Mg in ²³Mg umgewandelt, das wiederum schnell zu ²³Na zerfällt. Eine weitere Kernreaktion ist ²³Na(p,x) ¹⁸F. Insgesamt liegt nach Bestrahlung als Kationen-Endprodukt nun auch ¹⁸F vor, das in der ursprünglichen Lösung nicht vorhanden war. Das ¹⁸F kann mithilfe eines Ionenaustauschers entfernt werden, z.B. aus der Lösung, die nach Extraktion des ^{99m}Tc das ^{99m}Tc enthält oder aus der Lösung, die nach Extraktion des ^{99m}Tc das verbleibende Molybdat enthält und die wieder der ursprünglichen Lösung zugeführt wird. Dadurch wird vermieden, dass durch Bestrahlung von ¹⁸F und durch den Rückführungskreislauf eine Kette nur schwer kontrollierbarer Kernreaktionen ausgelöst würde.

In einer weiteren Ausführungsvariante umfasst das ¹⁰⁰Mo-Molybdat-Salz K₂¹⁰⁰MoO_{4,} wobei das Kationen-Endprodukt ⁴¹Ca umfasst. Das natürlich vorkommende ⁴¹K wird durch den Protonenstrahl in folgenden Kernreaktionen umgewandelt: ⁴¹K(p,n) ⁴¹Ca, ⁴¹K (p, γ) ⁴²Ca, ⁴¹K (p, αγ) ³⁸Ar. Das ebenfalls natürlich vorkommende ³⁹K wird durch den Protonenstrahl in folgenden Kernreaktionen umgewandelt: ³⁹K(p,d) ³⁸K, ^{39K}(p, γ) ⁴⁰Ca. ³⁸K zerfällt zu ³⁸Ar. Von den entstandenen Ca-Ionen ist lediglich das ⁴¹Ca instabil. Durch den Ionenaustauscher können alle Ionen entfernt werden. Eine Rückführung von ³⁸Ar ist unkritisch, da der Wechselwirkungsquerschnitt für die Wechselwirkung mit dem Protonstrahl in einem anderen Bereich liegt als der Wechselwirkungsquerschnitt für die ¹⁰⁰Mo(p,2n) ^{99m}Tc-Kernreaktion. Rückführung und Bestrahlung von ³⁸Ar erzeugt daher keine Kernreaktionskette mit unkontrollierbaren Endprodukten.

Die Vorrichtung zur Produktion von ^{99m}Tc, umfasst
- eine Lösung mit ¹⁰⁰Mo-Molybdat-Ionen,
- einen Beschleuniger zur Bereitstellung eines Protonenstrahls mit einer Energie die geeignet ist, bei Bestrahlung von ¹⁰⁰Mo-Molybdat-Ionen eine ¹⁰⁰Mo(p,2n) ^{99m}Tc-Kernreaktion zu induzieren, zur Bestrahlung der Lösung und zur Induktion einer ¹⁰⁰Mo (p, 2n) ^{99m}Tc-Kernreaktion,
- einer Extraktionsstufe zur Extraktion des ^{99m}Tc aus der Lösung.

In einer Ausführungsvariante ist die Lösung mit ¹⁰⁰Mo-Molybdat-Ionen eine Lösung eines ¹⁰⁰Mo-Molybdat-Salzes, wobei in der Lösung bei Bestrahlung mit dem Protonenstrahl bei den Kationen des ¹⁰⁰Mo-Molybdat-Salzes eine Kernreaktion induziert wird, welche in mindestens ein Kationen-Endprodukt mündet, und wobei die Vorrichtung zusätzlich eine der Extraktionsstufe nachgeschaltete Reinigungsstufe aufweist, in der das extrahierte ^{99m}Tc von Verunreinigungen durch das Kationen-Endprodukt reinigbar ist.

In einer Ausführungsvariante ist ein Kreislauf vorgesehen, mit dem die nach Extraktion des ^{99m}Tc verbleibenden gelösten ¹⁰⁰Mo-Molybdat-Ionen der bestrahlenden Lösung durch einen z.B. geschlossenen Kreislauf wieder zuführbar sind. Insbesondere können, wenn die Lösung mit ¹⁰⁰Mo-Molybdat-Ionen eine Lösung eines ¹⁰⁰Mo-Molybdat-Salzes ist, die Vorrichtung zusätzlich eine im Kreislauf zwischengeschaltete Reinigungsstufe aufweisen, in der das mindestens eine Kationen-Endprodukt vor Zuführung der verbleibenden gelösten ¹⁰⁰Mo-Molybdat-Ionen entfernt wird, insbesondere unter Verwendung eines Ionentauschers.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
Fig. 1 den Aufbau einer Vorrichtung zur Erzeugung von ^{99m}Tc aus einem Lithium-Molybdat-Salz,
Fig. 2 den Aufbau einer Vorrichtung zur Erzeugung von ^{99m}Tc aus einem Natrium-Molybdat-Salz,
Fig. 3 den Aufbau einer Vorrichtung zur Erzeugung von ^{99m}Tc aus einem Kalium-Molybdat-Salz.

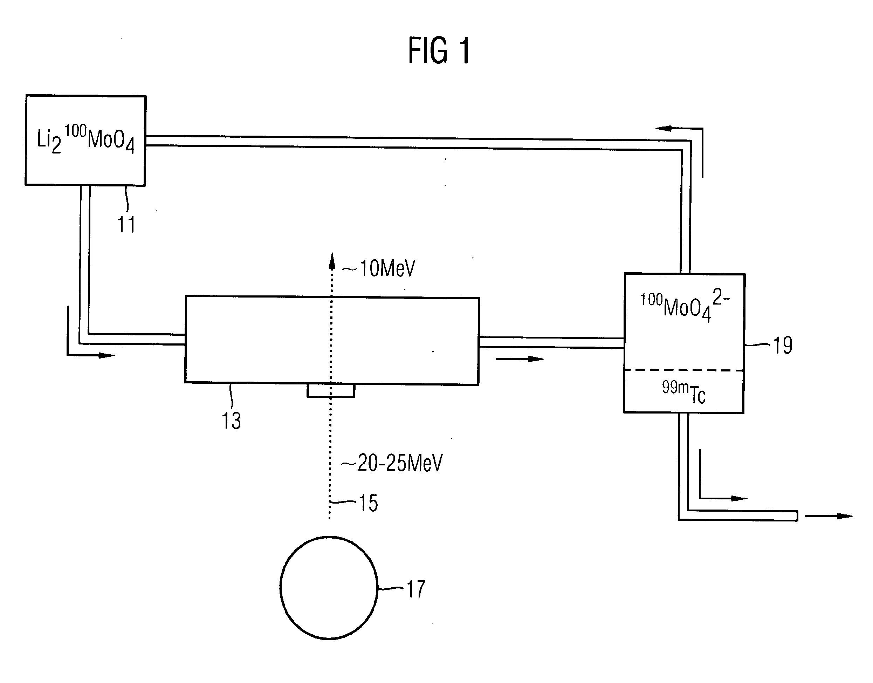

Gemäß der Ausführungsform von Fig. 1 wird zunächst eine wässrige Lösung 11 bereitgestellt, in welcher ⁶Li₂¹⁰⁰MoO₄ gelöst ist.

Die Lösung 11 wird anschließend zu einer Bestrahlungskammer 13 geführt, welche mit einem Protonenstrahl 15, der von einer Beschleunigereinheit 17 wie beispielsweise einem Zyklotron erzeugt wird, bestrahlt wird. Der Protonenstrahl 15 hat dabei bei Eintritt in die Bestrahlungskammer 13 eine Energie von 20 bis 25 MeV, und bei Austritt eine Energie von circa 10 MeV. In diesem Energiebereich wechselwirkt der Protonenstrahl 15 mit dem ¹⁰⁰Mo und wandelt dieses in einer Kernreaktion teilweise direkt in ^{99m}Tc um, anhand der Kernreaktion ¹⁰⁰Mo(p, 2n) ^{99m}Tc.

Durch Bestrahlung der ⁶Li-Ionen treten weiterhin folgende Kernreaktionen auf: ⁶Li(p, 3He) ⁴H wobei das ⁴H sofort zu Tritium zerfällt.

Die bestrahlte Lösung wird einer Kammer 19 zur Solventextraktion zugeführt, in der mithilfe von MEK (Methylethylketon) das ^{99m}Tc aus der wässrigen Lösung extrahiert wird. Das in MEK gelöste ^{99m}Tc kann dann weiterverarbeitet werden, beispielsweise in einem anschließenden Pharma-Modul (nicht gezeigt).

Die verbleibende Lösung des Molybdat-Salzes wird der ursprünglich bereitgestellten Lösung 11 wieder zugeführt.

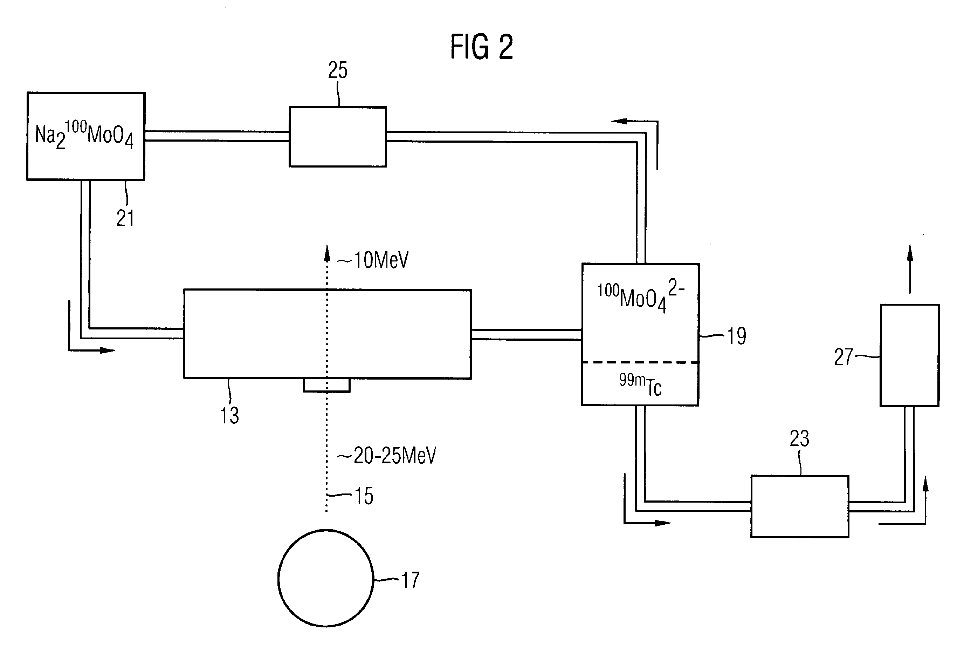

Die Ausführungsform von Fig. 2 unterscheidet sich von Fig. 1 dadurch, dass zunächst eine wässrige Lösung 21 bereitgestellt wird, in welcher Na₂¹⁰⁰MoO₄ gelöst ist.

Durch Bestrahlung der Na-Ionen treten folgende Kernreaktionen auf: ²³Na(p, n) ²³Mg und ²³Na(p, x) ¹⁸F. ²³Mg zerfällt wiederum in das stabile ²³Na. ¹⁸F hingegen ist radioaktiv.

Die bestrahlte Lösung wird einer Kammer 19 zur Solventextraktion zugeführt, in der mithilfe von MEK (Methylethylketon) das ^{99m}Tc aus der wässrigen Lösung extrahiert wird. Vor weiterer Verarbeitung können Verunreinigungen durch das ¹⁸F mithilfe eines ersten Ionenaustauschers 23 entfernt werden.

Ebenso kann das ¹⁸F mithilfe eines weiteren Ionenaustauschers 25 entfernt werden, bevor die nach Extraktion von ^{99m}Tc verbleibende Lösung des Molybdat-Salzes der ursprünglich bereitgestellten Lösung 21 wieder zugeführt wird.

Die extrahierte und von ¹⁸F gereinigte ^{99m}Tc-Lösung 27 kann dann z.B. einem anschließenden Pharma-Modul bereitgestellt werden.

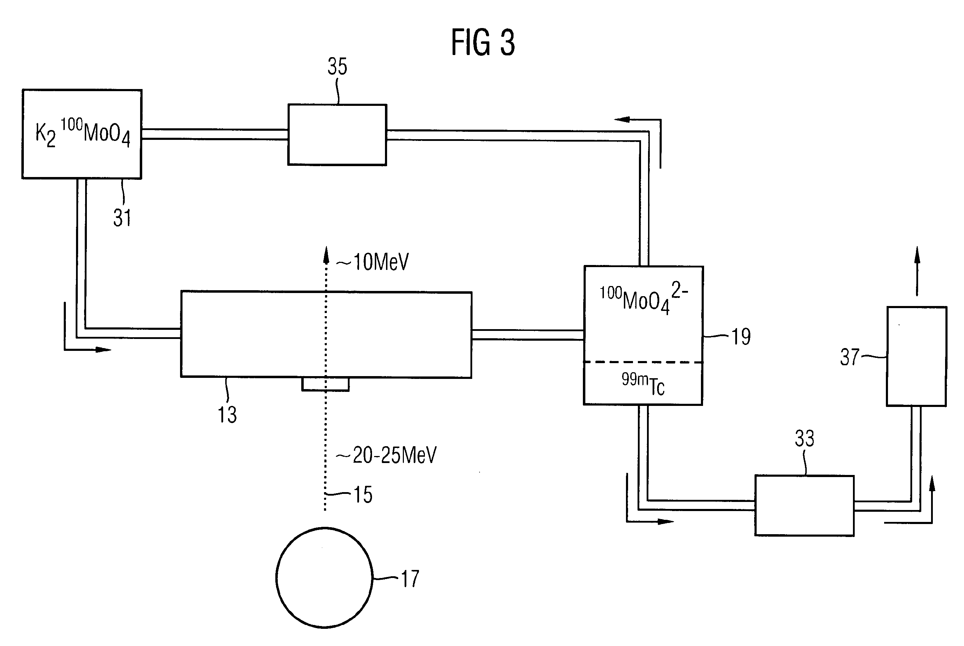

Die Ausführungsform von Fig. 3 unterscheidet sich von Fig. 1 dadurch, dass zunächst eine wässrige Lösung 31 bereitgestellt wird, in welcher K₂¹⁰⁰MoO₄ gelöst ist.

Durch Bestrahlung der Na-Ionen treten folgende Kernreaktionen auf: ⁴¹K(p, n) ⁴¹Ca, ⁴¹K(p, γ)42Ca, ⁴¹K(p, αγ) ³⁸Ar, ³⁹K (p,d) ³⁸K, ³⁹K(p, γ) ⁴⁰Ca. Von den entstehenden Kationen-Endprodukten ist lediglich das ⁴¹Ca instabil.

Die bestrahlte Lösung wird einer Kammer 19 zur Solventextraktion zugeführt, in der mithilfe von MEK (Methylethylketon) das ^{99m}Tc aus der wässrigen Lösung extrahiert wird.

Vor weiterer Verarbeitung können Verunreinigungen durch das ⁴¹Ca mithilfe eines ersten Ionenaustauschers 33 entfernt werden.

Ebenso kann das ⁴¹Ca und die anderen Ca-Ionen mithilfe eines weiteren Ionenaustauschers 35 entfernt werden, bevor die nach Extraktion von ^{99m}Tc verbleibende Lösung des Molybdat-Salzes der ursprünglich bereitgestellten Lösung 31 wieder zugeführt wird.

Die extrahierte und von ⁴¹Ca gereinigte ^{99m}Tc -Lösung kann dann z.B. in einer Trockeneinheit 37 getrocknet und einem anschließenden Pharma-Modul (nicht gezeigt) bereitgestellt werden.

### Bezugszeichenliste

- 11, 21, 31: wässrige Lösung
- 13: Bestrahlungskammer
- 15: Protonenstrahl
- 17: Beschleunigereinheit
- 19: Kammer zur Solventextraktion
- 23, 33: erster Ionenaustauscher
- 25, 35: weitere Ionenaustauscher
- 27: gereinigte ^{99m}Tc-Lösung
- 37: Trockeneinheit

## Patentansprüche

1. Verfahren zur Produktion von ^{99m}Tc, umfassend folgende Schritte:
- Bereitstellen einer Lösung (11, 21, 31) mit ¹⁰⁰Mo-Molybdat-Ionen,
- Bereitstellen eines Protonenstrahls (15) mit einer Energie, die geeignet ist, bei Bestrahlung von ¹⁰⁰Mo-Molybdat-Ionen eine ¹⁰⁰Mo(p, 2n) ^{99m}Tc-Kernreaktion zu induzieren,
- Bestrahlen der Lösung mit dem Protonenstrahl (15) und Induktion einer ¹⁰⁰Mo(p, 2n) ^{99m}Tc-Kernreaktion,
- Anwenden eines Extraktionsverfahrens zur Extraktion des ^{99m}Tc aus der Lösung.

2. Verfahren nach Anspruch 1, wobei
das Extraktionsverfahren ein Solventextraktionsverfahren, insbesondere unter Verwendung von Methylethylketon, ist.

3. Verfahren nach Anspruch 1 oder 2, wobei
die nach Extraktion des ^{99m}Tc verbleibenden gelösten ¹⁰⁰Mo-Molybdat-Ionen der zu bestrahlenden Lösung wieder zugeführt werden.

4. Verfahren nach einem der vorhergehenden Ansprüche, wobei die Lösung mit ¹⁰⁰Mo-Molybdat-Ionen eine Lösung eines ¹⁰⁰Mo-Molybdat-Salzes (11, 21, 31) ist, wobei in der Lösung (11, 21, 31) durch Bestrahlen mit dem Protonenstrahl bei den Kationen des ¹⁰⁰Mo-Molybdat-Salzes eine Kernreaktion induziert wird, welche in mindestens ein Kationen-Endprodukt mündet.

5. Verfahren nach Anspruch 4, wobei
nach Extraktion des ^{99m}Tc die verbleibenden, gelösten ¹⁰⁰Mo-Molybdat-Ionen der bestrahlenden Lösung wieder zugeführt werden und das mindestens eine Kationen-Endprodukt vor Zuführung entfernt wird, insbesondere unter Verwendung eines Ionentauschers (25, 35).

6. Verfahren nach Anspruch 4 oder 5, wobei nach Extraktion des ^{99m}Tc aus der Lösung das extrahierte ^{99m}Tc von Verunreinigungen durch das Kationen-Endprodukt gereinigt wird, insbesondere unter Verwendung eines Ionenaustauschers (23, 33).

7. Verfahren nach einem der Ansprüche 4 bis 6, wobei das ¹⁰⁰Mo-Molybdat-Salz ⁶Li₂¹⁰⁰MoO₄ umfasst, und wobei das mindestens eine Kationen-Endprodukt ³H umfasst.

8. Verfahren nach einem der Ansprüche 4 bis 7, wobei das ¹⁰⁰Mo-Molybdat-Salz Na₂¹⁰⁰MoO₄ umfasst, und wobei das Kationen-Endprodukt ¹⁸F umfasst.

9. Verfahren nach einem der Ansprüche 4 bis 8, wobei das ¹⁰⁰Mo-Molybdat-Salz K₂¹⁰⁰MoO₄ umfasst, und wobei das Kationen-Endprodukt Ca-Ionen umfasst.

10. Vorrichtung zur Produktion von ^{99m}Tc, umfassend
- eine Lösung (11, 21, 31) mit ¹⁰⁰Mo-Molybdat-Ionen,
- einen Beschleuniger zur Bereitstellung eines Protonenstrahls mit einer Energie die geeignet ist, bei Bestrahlung von ¹⁰⁰Mo-Molybdat-Ionen eine ¹⁰⁰Mo(p, 2n) ^{99m}Tc-Kernreaktion zu induzieren, zur Bestrahlung der Lösung und zur Induktion einer ¹⁰⁰Mo (p, 2n) ^{99m}Tc-Kernreaktion,
- einer Extraktionsstufe (19) zur Extraktion des ^{99m}Tc aus der Lösung.

11. Vorrichtung nach Anspruch 10, wobei die nach Extraktion des ^{99m}Tc verbleibenden gelösten ¹⁰⁰Mo-Molybdat-Ionen der zu bestrahlenden Lösung (11, 21, 31) durch einen Kreislauf wieder zuführbar sind.

12. Vorrichtung nach einem der Ansprüche 10 oder 11,
wobei die Lösung mit ¹⁰⁰Mo-Molybdat-Ionen eine Lösung (11, 21, 31) eines ¹⁰⁰Mo-Molybdat-Salzes ist, wobei in der Lösung (11, 21, 31) bei Bestrahlung mit dem Protonenstrahl (15) bei den Kationen des ¹⁰⁰Mo-Molybdat-Salzes eine Kernreaktion induzierbar ist, welche in mindestens ein Kationen-Endprodukt mündet.

13. Vorrichtung nach Anspruch 12,
wobei die Vorrichtung zusätzlich eine der Extraktionsstufe (19) nachgeschaltete erste Reinigungsstufe (23, 33) aufweist, in der das extrahierte ^{99m}Tc von Verunreinigungen durch das Kationen-Endprodukt reinigbar ist.

14. Vorrichtung nach Anspruch 12 oder 13,
wobei die Vorrichtung zusätzlich eine zweite Reinigungsstufe (25, 35) aufweist, in der das mindestens eine Kationen-Endprodukt vor Zuführung der verbleibenden gelösten ¹⁰⁰Mo-Molybdat-Ionen zu der zu bestrahlenden Lösung (11, 21, 31) entfernt wird, insbesondere unter Verwendung eines Ionentauschers.

## Claims

1. Method for producing ^{99m}Tc, comprising the following steps:
- providing a solution (11, 21, 31) with ¹⁰⁰Mo-molybdate ions,
- providing a proton beam (15) with an energy suitable for inducing a ¹⁰⁰Mo(p, 2n) ^{99m}Tc nuclear reaction when ¹⁰⁰Mo-molybdate ions are irradiated,
- irradiating the solution with the proton beam (15) and inducing a ¹⁰⁰Mo(p, 2n) ^{99m}Tc nuclear reaction,
- applying an extraction method for extracting the ^{99m}Tc from the solution.

2. Method according to Claim 1, wherein
the extraction method is a solvent extraction method, more particularly using methyl ethyl ketone.

3. Method according to Claim 1 or 2, wherein
the dissolved ¹⁰⁰Mo-molybdate ions remaining after the ^{99m}Tc extraction are returned to the solution to be irradiated.

4. Method according to one of the preceding claims, wherein the solution with ¹⁰⁰Mo-molybdate ions is a solution of a ¹⁰⁰Mo-molybdate salt (11, 21, 31), wherein a nuclear reaction which leads to at least one cation end product is induced in the solution (11, 21, 31) by irradiation with the proton beam at the cations of the ¹⁰⁰Mo-molybdate salt.

5. Method according to Claim 4, wherein,
after extracting the ^{99m}Tc, the remaining, dissolved ¹⁰⁰Mo-molybdate ions are returned to the irradiating solution and the at least one cation end product is removed before the supply, more particularly by using an ion exchanger (25, 35).

6. Method according to Claim 4 or 5, wherein, after extracting the ^{99m}Tc from the solution, the extracted ^{99m}Tc is cleansed of impurities resulting from the cation end product, more particularly by using an ion exchanger (23, 33).

7. Method according to one of Claims 4 to 6, wherein the ¹⁰⁰Mo-molybdate salt comprises ⁶Li₂¹⁰⁰MoO4, and wherein the at least one cation end product comprises ³H.

8. Method according to one of Claims 4 to 7, wherein the ¹⁰⁰Mo-molybdate salt comprises Na₂¹⁰⁰MoO₄, and wherein the cation end product comprises ¹⁸F.

9. Method according to one of Claims 4 to 8, wherein the ¹⁰⁰Mo-molybdate salt comprises K₂¹⁰⁰MoO₄, and wherein the cation end product comprises Ca ions.

10. Device for producing ^{99m}Tc, comprising
- a solution (11, 21, 31) with ¹⁰⁰Mo-molybdate ions,
- an accelerator for providing a proton beam with an energy suitable for inducing a ¹⁰⁰Mo(p, 2n) ^{99m}Tc nuclear reaction when ¹⁰⁰Mo-molybdate ions are irradiated, for irradiating the solution and for inducing a ¹⁰⁰Mo(p, 2n) ^{99m}Tc nuclear reaction,
- an extraction stage (19) for extracting the ^{99m}Tc from the solution.

11. Device according to Claim 10, wherein the dissolved ¹⁰⁰Mo-molybdate ions remaining after the ^{99m}Tc extraction can be returned to the solution (11, 21, 31) to be irradiated by a loop.

12. Device according to either of Claims 10 and 11, wherein the solution with ¹⁰⁰Mo-molybdate ions is a solution (11, 21, 31) of a ¹⁰⁰Mo-molybdate salt, wherein a nuclear reaction which leads to at least one cation end product can be induced in the solution (11, 21, 31) by irradiation with the proton beam (15) at the cations of the ¹⁰⁰Mo-molybdate salt.

13. Device according to Claim 12,
wherein the device additionally has a first cleaning stage (23, 33) downstream of the extraction stage (19), in which cleaning stage the extracted ^{99m}Tc can be cleansed of impurities resulting from the cation end product.

14. Device according to Claim 12 or 13,
wherein the device additionally has a second cleaning stage (25, 35), in which the at least one cation end product is removed, more particularly by using an ion exchanger, before the remaining, dissolved ¹⁰⁰Mo-molybdate ions are supplied to the solution (11, 21, 31) to be irradiated.

## Revendications

1. Procédé de production de ^{99m}Tc, comprenant les étapes suivantes:
- mise à disposition d'une solution (11, 21, 31) avec des ions molybdate ¹⁰⁰Mo,
- mise à disposition d'un faisceau de protons (15) avec une énergie qui est apte à induire une réaction nucléaire ¹⁰⁰Mo(p, 2n) ^{99m}Tc lors de l'irradiation d'ions molybdate ¹⁰⁰Mo,
- irradiation de la solution avec le faisceau de protons (15) et induction d'une réaction nucléaire ¹⁰⁰Mo(p, 2n) ^{99m}Tc,
- application d'un procédé d'extraction pour l'extraction du ^{99m}Tc hors de la solution.

2. Procédé selon la revendication 1, dans lequel le procédé d'extraction est un procédé d'extraction par solvant, en particulier avec utilisation de méthyléthylcétone.

3. Procédé selon la revendication 1 ou 2, dans lequel les ions molybdate ¹⁰⁰Mo dissous restants après l'extraction de ^{99m}Tc sont de nouveau envoyés dans la solution à irradier.

4. Procédé selon l'une quelconque des revendications précédentes, dans lequel la solution avec des ions molybdate ¹⁰⁰Mo est une solution d'un sel de molybdate ¹⁰⁰Mo (11, 21, 31), dans lequel on induit dans la solution (11, 21, 31), par irradiation avec un faisceau de protons (15), dans les cations du sel de molybdate ¹⁰⁰Mo une réaction nucléaire qui débouche sur au moins un produit final de cations.

5. Procédé selon la revendication 4, dans lequel, après l'extraction du ^{99m}Tc, les ions molybdate ¹⁰⁰Mo dissous restants sont de nouveau envoyés à la solution à irradier et ledit au moins un produit final de cations est retiré avant cet envoi, en particulier par utilisation d'un échangeur d'ions (25, 35).

6. Procédé selon la revendication 4 ou 5, dans lequel, après l'extraction du ^{99m}Tc hors de la solution, le ^{99m}Tc extrait est débarrassé des impuretés par le produit final de cations, en particulier par utilisation d'un échangeur d'ions (23, 33).

7. Procédé selon l'une quelconque des revendications 4 à 6, dans lequel le sel de molybdate ¹⁰⁰Mo comprend du ⁶Li₂¹⁰⁰MoO₄, et dans lequel le produit final de cations comprend du ³H.

8. Procédé selon l'une quelconque des revendications 4 à 7, dans lequel le sel de molybdate ¹⁰⁰Mo comprend du Na₂¹⁰⁰MoO₄, et dans lequel le produit final de cations comprend du ¹⁸F.

9. Procédé selon l'une quelconque des revendications 4 à 8, dans lequel le sel de molybdate ¹⁰⁰Mo comprend du K₂¹⁰⁰MoO₄, et dans lequel le produit final de cations comprend des ions Ca.

10. Dispositif de production de ^{99m}Tc, comprenant:
- une solution (11, 21, 31) avec des ions molybdate ¹⁰⁰Mo,
- un accélérateur pour la mise à disposition d'un faisceau de protons avec une énergie qui est apte, lors de l'irradiation d'ions molybdate ¹⁰⁰Mo, à induire une réaction nucléaire ¹⁰⁰Mo(p, 2n) ^{99m}Tc, pour l'irradiation de la solution et pour l'induction d'une réaction nucléaire ¹⁰⁰Mo (p, 2n) ^{99m}Tc,
- un étage d'extraction (19) pour l'extraction du ^{99m}Tc hors de la solution.

11. Dispositif selon la revendication 10, dans lequel les ions molybdate ¹⁰⁰Mo dissous restants après l'extraction du ^{99m}Tc peuvent être de nouveau envoyés à la solution à irradier (11, 21, 31) par un circuit.

12. Dispositif selon une des revendications 10 ou 11, dans lequel la solution avec des ions molybdate ^{99m}Tc est une solution (11, 21, 31) d'un sel de molybdate ¹⁰⁰Mo, dans lequel il est possible d'induire dans la solution (11, 21, 31), par irradiation avec le faisceau de protons (15) dans les cations du sel de molybdate ¹⁰⁰Mo, une réaction nucléaire qui débouche sur au moins un produit final de cations.

13. Dispositif selon la revendication 12, dans lequel le dispositif présente en plus un premier étage (23, 33) disposé en aval de l'étage d'extraction (19), dans lequel le ^{99m}Tc extrait peut être débarrassé d'impuretés par le produit final de cations.

14. Dispositif selon la revendication 12 ou 13, dans lequel le dispositif présente en plus un deuxième étage de nettoyage (25, 35), dans lequel ledit au moins un produit final de cations est retiré avant l'envoi des ions molybdate ¹⁰⁰Mo dissous restants à la solution à irradier (11, 21, 31), en particulier par utilisation d'un échangeur d'ions.
